# FASCICULE DE BREVET EUROPEEN

(11) **EP 3 370 820 B1**
(45) Date de publication et mention de la délivrance du brevet: **25.03.2020**
(21) Numéro de dépôt: 16806252.9
(22) Date de dépôt: 04.11.2016
(51) Int. Cl.: A61N 1/30, A61N 1/32

(54) **DISPOSITIF D'APPLICATION D'UN PRODUIT A DISTRIBUER SUR LA PEAU D'UN UTILISATEUR PAR IONTOPHORESE COMPRENANT DES MOYENS DE MESURE D'ABSENCE DE PRODUIT ET PROCEDE CORRESPONDANT**
VORRICHTUNG ZUM AUFTRAGEN EINES DURCH IONTOPHORESE AUF DIE HAUT EINES BENUTZERS ABZUGEBENDEN PRODUKTS MIT MITTELN ZUR MESSUNG DER FEHLENDEN PRODUKTMENGE UND ENTSPRECHENDES VERFAHREN
DEVICE FOR APPLYING A PRODUCT TO BE DISPENSED ON THE SKIN OF A USER BY IONTOPHORESIS COMPRISING MEANS FOR MEASURING THE ABSENCE OF PRODUCT AND CORRESPONDING METHOD

(30) Priorité: 06.11.2015 FR 1560680
(43) Date de publication de la demande: 12.09.2018
(73) Titulaire: SEB S.A., 69130 Ecully (FR)
(72) Inventeur: MANDICA, Franck, 69340 Francheville (FR); SABATTIER, Johan, 69440 Mornant (FR)
(74) Mandataire: Bourrières, Patrice
(86) Numéro de dépôt international: PCT/FR2016/052869
(87) Numéro de publication internationale: WO 2017/077256

(56) Documents cités:
- EP-A2- 0 942 278
- WO-A2-02/085451
- FR-A1- 2 980 370
- US-A1- 2002 161 323

## Description

### 1. Domaine de l'invention

L'invention concerne le domaine des dispositifs permettant l'application d'un produit cosmétique ou thérapeutique sur la peau d'un utilisateur. En particulier, elle vise les dispositifs utilisant le principe de la iontophorèse pour améliorer la délivrance d'un principe actif d'un produit à travers les différentes couches de la peau.

### 2. Art antérieur

Il est connu un dispositif d'application d'un produit à distribuer sur la peau par iontophorèse destiné à améliorer la délivrance d'un principe actif d'un produit à travers la peau. Un tel dispositif est connu du document WO 2013/118114 lequel comprend un réservoir pour le produit à appliquer, au moins une première électrode propre à générer un champ électrique de manière à permettre la pénétration d'un principe actif du produit dans la peau, un circuit électronique de commande, et des moyens de mesure reliés au circuit de commande. Ces moyens de mesure sont propres à mesurer des paramètres de la peau ou de la substance, de la quantité de produit qui reste dans le réservoir ou du temps écoulé pour générer une distribution automatique de produit sur la peau.

L'un des problèmes liés à ce dispositif d'application de produit à distribuer réside dans le fait qu'il s'agit là d'un système complexe faisant intervenir tous types de moyens de mesure. Les mesures effectuées au niveau de la peau ne sont pas précises car la peau réagit différemment d'un utilisateur à l'autre. Par exemple, quand il s'agit d'une mesure d'impédance, celle de la peau varie tout particulièrement en fonction de son hydratation. D'autre part, un circuit de temporisation ne permet pas d'avoir une gestion précise du produit à distribuer et de la consommation de celui-ci. Enfin, il n'y aucun enseignement sur la manière de gérer ces différents paramètres pour la distribution de produit.

### 3. Objectifs de l'invention

L'invention a notamment pour objectif de pallier tout ou partie des inconvénients de l'art antérieur.

Un objectif de l'invention est de fournir un dispositif d'application d'un produit à distribuer par iontophorèse qui permet de savoir lorsqu'il y a absence de produit de sorte que le traitement ne soit pas interrompu tout en protégeant la peau de l'utilisateur.

### 4. Résumé de l'invention

Ces objectifs sont atteints grâce à un dispositif d'application d'un produit à distribuer sur une peau d'un utilisateur par iontophorèse comprenant:
- au moins un moyen de stockage du produit à distribuer;
- au moins une cavité de distribution propre à recevoir du produit à distribuer ;
- au moins une première électrode pour iontophorère ;
- un circuit électronique de commande;
- des moyens de mesure d'un paramètre physique ou physico-chimique reliés au circuit électronique de commande et configurés de manière à envoyer un signal indicatif de mesure de paramètre,
la première électrode pour iontophorèse et les moyens de mesures sont installés dans la cavité de distribution, le circuit électronique de commande étant configuré pour comparer la valeur mesurée du paramètre physique ou physico-chimique dans la cavité à une valeur seuil de référence de façon à détecter s'il y a absence du produit dans la cavité.

Cette solution permet de résoudre les problèmes précités. Ainsi, le dispositif d'application de produit permet d'avoir en permanence une information sur l'absence du produit dans la cavité de distribution et donc sur la peau de l'utilisateur. Le dispositif ou l'utilisateur ayant connaissance de cette information déclenche une distribution du produit vers la peau de l'utilisateur. Le fait de savoir qu'il n'y a plus de produit dans la cavité de distribution évite les microlésions survenant lors d'une application de courant prolongée et/ou de frottement du dispositif sur la peau sans le produit et donc sans traitement possible. Aussi, le moyen de mesure d'un paramètre physique ou physico-chimique dans la cavité de distribution permet une mesure plus précise que celles effectuées directement sur la peau d'un utilisateur.

Selon une caractéristique de l'invention, le dispositif comprend au moins une deuxième électrode pour iontophorèse agencée dans la cavité de distribution. Ainsi, la circulation du courant reste localisée entre la première électrode et la deuxième électrode dans la zone à traiter alors que dans un dispositif dit monopolaire le courant traverse une grande partie du corps (par exemple main, visage et cerveau si une contre électrode est sur le corps de l'appareil au contact de la paume de la main).

Selon une caractéristique de l'invention, le circuit électronique de commande est configuré, s'il détecte l'absence de produit dans la cavité de distribution, pour générer un signal de commande propre à déclencher la distribution de produit dans la cavité de distribution. De la sorte, la distribution de produit est automatique et l'utilisateur peut continuer à traiter les différentes zones de la peau sans se préoccuper d'autres commandes du dispositif.

Selon encore une autre caractéristique de l'invention, le circuit électronique de commande est relié aux première et deuxième électrodes pour iontophorèse et est configuré pour générer un courant électrique propre à circuler côté peau entre les première et deuxième électrodes pour iontophorèse. Ainsi, il est possible de gérer la circulation de courant dans les électrodes de iontophorèse afin d'une part de ne pas perturber les moyens de mesure et d'autre part de gérer la consommation énergétique/électrique du dispositif d'application.

Selon une autre caractéristique de l'invention, le dispositif comprend des moyens d'alerte connectés au circuit électronique de commande, le circuit électronique de commande étant configuré, s'il détecte l'absence de produit dans la cavité de distribution, pour générer un signal de commande propre à avertir l'utilisateur de l'absence de produit dans la cavité. L'utilisateur est ainsi averti de l'absence de produit dans la cavité et peut décider de lui-même de continuer ou de ne pas continuer le traitement ou le soin des zones de traitement de la peau.

Selon une autre caractéristique de l'invention, le dispositif comprend au moins un moyen d'extraction du produit à distribuer à partir du moyen de stockage. En particulier, ce moyen d'extraction peut être à commande manuelle ou motorisée. Ainsi, lorsque l'utilisateur est averti du manque de produit, celui-ci peut actionner le moyen d'extraction pour extraire le produit du moyen de stockage autrement, l'extraction du produit du moyen de stockage est automatisée.

Selon un premier mode de réalisation de l'invention, les moyens de mesure comprennent un moyen de mesure d'impédance du produit. Un tel moyen de mesure d'impédance permet d'associer un seuil de détection au type de produit à distribuer, qui est plus ou moins conducteur selon sa composition.

Selon une caractéristique de ce mode de réalisation, le moyen de mesure d'impédance comprend au moins une paire d'électrodes pour la mesure d'impédance, disposée dans la cavité de distribution et entre lesquelles circule un courant électrique.

Selon un deuxième mode de réalisation de l'invention, les moyens de mesure comprennent un moyen de mesure optique. Un tel moyen de mesure optique permet, en particulier, de s'affranchir des problématiques d'oxydation des électrodes de mesure décrites dans le mode de réalisation précédent si elles sont utilisées au contact de produits oxydants ou corrosifs par exemple.

Selon une caractéristique de ce deuxième mode de réalisation, le moyen de mesure optique comprend au moins un émetteur de lumière diffusant une lumière à travers au moins une des cavités de distribution et au moins un récepteur de lumière placé en regard de l'émetteur

Selon une caractéristique de l'invention, la première et la deuxième électrodes pour iontophorèse sont séparées par une zone inter-électrode laquelle comprend une surface d'application du produit sur la peau. De la sorte, les première et deuxième électrodes pour iontophorèse sont maintenues à distance l'une de l'autre. Par ailleurs, cela permet de générer un courant de plus faible intensité dans celles-ci pour des mesures de sécurité.

Selon encore une autre caractéristique de l'invention, les première et deuxième électrodes pour iontophorèse sont situées dans un même plan. Cette configuration permet de réduire la distance parcourue par le courant électrique entre les première et deuxième électrodes pour iontophorèse par rapport à un dispositif comportant une électrode pour iontophorèse propre à être en contact avec la zone à traiter ou soigner et une deuxième électrode pour iontophorèse sur une poignée du dispositif. D'autre part, la pénétration des principes actifs à travers la peau de l'utilisateur est maitrisée. De la sorte, il est possible de cibler la zone de la peau à traiter et limiter les courants de fuite.

Selon une caractéristique de l'invention, la surface d'application est située dans un plan parallèle audit plan des première et deuxième électrodes, le plan des première et deuxième électrodes pour iontophorèse et le plan de la zone inter-électrode étant situés à une distance prédéterminée. Ainsi, la disposition des première et deuxième électrodes en retrait de la zone inter-électrode permet d'éviter un contact direct de celles-ci avec la peau de l'utilisateur ce qui empêche les irritations ou picotements électriques. Cela permet également de réduire la quantité de produit pouvant séjourner/s'accumuler entre le plan des première et deuxième électrodes et le plan de la zone inter-électrode. En outre, cette configuration permet de favoriser le passage de courant dans la peau et non pas à travers la formule située sur le plan des première et deuxième électrodes pour iontophorèse.

Selon une caractéristique de l'invention, le dispositif comprend un corps comportant le moyen de stockage et une tête d'application montée sur le corps. Cette configuration permet de favoriser la compacité du dispositif d'application et la simplicité d'utilisation et de manipulation.

Selon encore une autre caractéristique de l'invention, la première électrode et la deuxième électrode pour iontophorèse sont agencées dans la tête d'application de sorte à fournir un dispositif compact et éviter les courants de fuite.

Selon encore une autre caractéristique de l'invention, les première et deuxième électrodes pour iontophorèse et la paire d'électrodes pour la mesure d'impédance sont indépendantes de manière à ne pas créer d'interférences dans leurs fonctionnements.

Afin de permettre un approvisionnement rapide en produit à distribuer, de fournir un dispositif compact, facile à entretenir et à manipuler, le moyen de stockage du produit à distribuer comprend une cartouche connectée de manière amovible au corps du dispositif d'application.

L'invention concerne également un procédé de détection de l'absence de produit à distribuer dans une cavité de distribution d'un dispositif d'application du produit selon l'une quelconque des caractéristiques susmentionnées. Le procédé comprenant les étapes suivantes
- mesure par les moyens de mesure d'une valeur d'un paramètre physique ou physico-chimique dans la cavité de distribution;
- transmission de cette valeur mesurée au circuit électronique de commande ;
- comparaison, par le circuit électronique de commande, de cette valeur mesurée de paramètre physique ou physico-chimique avec une valeur seuil de référence ;
- détection, par le circuit électronique de commande, de l'absence de produit dans la cavité de distribution.

### 5. Liste des figures

D'autres caractéristiques et avantages innovants ressortiront de la description ci-après, fournie à titre indicatif et nullement limitatif, en référence aux dessins annexés, dans lesquels :
- La figure 1 est une vue en perspective d'un dispositif d'application de produit à distribuer sur la peau d'un utilisateur par iontophorèse selon l'invention;
- La figure 2 est une vue en en coupe transversale longitudinale du dispositif d'application de produit selon le plan de coupe A-A de la figure 1.
- La figure 3 est une vue de dessus et en perspective d'un exemple de tête d'application d'un dispositif d'application de produit selon l'invention ;
- La figure 4 est une vue de dessous de l'exemple de tête d'application illustrée sur la figure 3 ;
- La figure 5 est une vue en perspective et éclatée d'un exemple de tête d'application d'un dispositif d'application selon l'invention.
- Les figures 6 et 7 illustrent, de manière schématique et plus en détail, une vue en coupe de deux exemples de tête d'application selon l'invention ;
- Les figures 8 à 10 représentent de manière schématique des modes de réalisation de l'agencement de première et deuxième électrodes dans la tête d'application ; et
- Les figures 11 à 13 représentent un exemple de moyen d'isolation électrique à interposer entre la première électrode et la deuxième électrode;
- La figure 14 est une vue en coupe partielle et de détail d'un exemple de tête d'application montée et connectée sur un corps de dispositif d'application lequel comprend une cartouche selon l'invention ;
- La figure 15 est un exemple de schéma bloc simplifié représentant les communications électriques entre un circuit électronique de commande, un générateur de courant et les première et deuxième électrodes ;
- La figure 16 représente un mode de réalisation de l'invention sur laquelle est représenté un exemple de réalisation de la tête d'application en éclatée avec des moyens de mesure ;
- La figure 17 est vue en coupe de la tête d'application du mode de réalisation illustré en figure 16 ;
- La figure 18 est une vue partielle et en perspective d'une tête d'application selon l'invention comprenant des moyens de mesure suivant un exemple de réalisation ;
- La figure 19 représente une vue partielle et en perspective d'une tête d'application selon l'invention avec des moyens de mesure suivant un autre exemple de réalisation ;
- La figure 20 illustre une vue de dessus et en perspective d'une portion de connexion du corps du dispositif destinée à se connecter avec une tête d'application selon l'invention ; et,
- La figure 21 est un exemple de schéma bloc représentant les différentes communications du circuit électronique de commande entre les moyens de mesure de paramètre physique ou physico-chimique dans la cavité de distribution, de moyen d'alerte, du générateur de courant et du moyen d'extraction du produit à distribuer.

### 6. Description détaillée

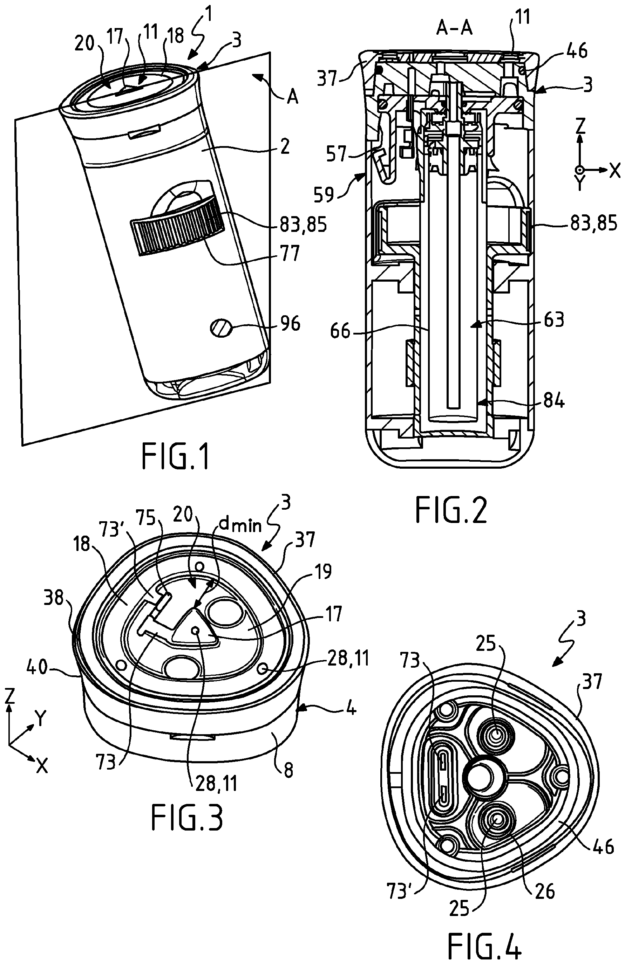

En référence à la figure 1, le dispositif 1 d'application d'un produit à distribuer sur la peau d'un utilisateur par iontophorèse selon l'invention est destiné au soin cosmétique et/ou thérapeutique de la peau de l'utilisateur. Le dispositif 1 ici utilise le principe d'iontophorèse, un principe du type électrophorèse pour favoriser et faciliter la pénétration du produit et de ses principes actifs à travers la peau de l'utilisateur. L'iontophorèse, ici, est l'application d'un champ électrique à travers la peau agissant comme une force motrice pour permettre le déplacement des ions du produit. La peau est traitée de manière non invasive.

Le dispositif comprend un corps 2 formant un organe de préhension du dispositif 1 et une tête d'application 3 montée sur le corps 2. Tel que visible à la figure 2, le dispositif comprend au moins un moyen de stockage 84 du produit à distribuer sur la peau. De manière avantageuse, mais non limitativement, le corps 2 comprend le moyen de stockage 84 du produit à distribuer. Quant à la tête d'application 3 celle-ci comprend au moins un moyen de distribution 11 du produit à distribuer sur la peau de l'utilisateur.

On entend par le terme « produit » au sens de l'invention, un produit se présentant sous forme d'un fluide tel qu'un liquide et/ou une composition aqueuse. Le produit comprend des principes actifs aptes à soigner ou traiter via la peau.

En référence aux figures 2 à 7, la tête d'application 3 est montée sur le corps 2 de manière amovible de sorte à permettre un remplacement de celle-ci en cas de détérioration et/ou d'endommagement, et aussi pour faciliter son nettoyage. La tête d'application 3 telle qu'illustrée sur les figures 3 et 4 comprend une interface supérieure destinée à être orientée vers la peau de l'utilisateur et une interface inférieure opposée destinée à être connectée avec le corps 2 du dispositif d'application de produit.

Pour faciliter la compréhension de l'invention, nous considérons que la tête d'application 3 s'étend suivant un axe longitudinal Z ici vertical. Est également représenté un axe horizontal X qui est perpendiculaire à l'axe longitudinal Z vertical et à un axe transversal Y de sorte que ces trois axes X, Y, Z forment un repère orthogonal direct tel qu'illustré sur les figures 2 et 3 par exemple. Les termes « inférieur », « supérieur », « haut », « bas », « dessus », et « dessous » sont définis par rapport à l'axe longitudinal vertical Z et le terme « latéral » est défini par rapport à l'axe horizontal X.

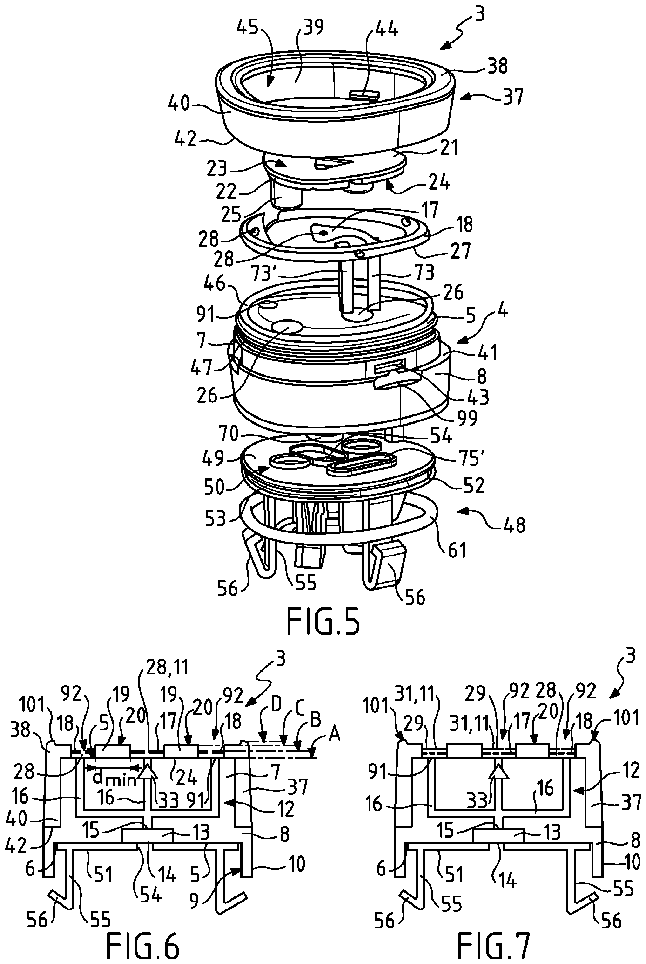

Plus précisément illustrée sur les figures 5, 6 et 7, la tête d'application 3 comprend une embase 4 comportant une surface supérieure 5 et une surface inférieure 6 reliées par une paroi 7. Cette paroi 7 est prolongée par une jupe latérale 8 qui s'étend vers le bas, selon l'axe Z, à partir de la périphérie de la paroi 7. La jupe latérale 8 présente une surface interne 9 et une surface externe 10 opposée suivant sa longueur. L'embase 4 présente une section générale sensiblement triangulaire. Bien entendu, l'embase 4 peut présenter une section circulaire ou autre dès lors que celle-ci soit compatible avec le corps du dispositif 1 d'application.

Sur les figures 6 et 7, l'embase 4 comporte également un circuit de distribution 12 du produit à distribuer agencé entre la surface supérieure 5 et la surface inférieure 6. En particulier, le circuit de distribution 12 comporte, à l'endroit de la surface inférieure 6 de l'embase 4, un réservoir tampon 13 destiné à recevoir, voire, collecter du produit en provenance du corps 2 du dispositif 1 d'application. Le réservoir tampon 13 présente une entrée 14 recevant le produit à distribuer du corps 2 et une sortie 15 permettant la circulation du produit vers le moyen de distribution 11 du produit sur la peau. Pour permettre la circulation du produit vers le moyen de distribution 11, le circuit de distribution 12 comporte des canaux de distribution 16 reliant le moyen de distribution 11 du produit et le réservoir tampon 13. Les canaux de distribution 16 sont formés dans la paroi 7 de l'embase 4. Ceux-ci débouchent sur la surface supérieure 5 de l'embase 4 par des orifices de distributions 91 et permettent en particulier de relier la sortie 15 du réservoir tampon 13 avec ces orifices de distribution 91. De manière avantageuse, mais non limitativement, chaque orifice de distribution 91 communique fluidiquement avec un canal de distribution 16 du circuit de distribution 12.

Le dispositif d'application comprend au moins une première électrode 17 propre à générer un courant électrique de manière à permettre la pénétration d'un principe actif du produit dans la peau. Dans le cadre de l'invention, le dispositif comprend au moins une deuxième électrode 18 pour iontophorèse. Ces première et deuxième électrodes 17, 18 sont installées chacune dans au moins une cavité de distribution 92 que comprend le dispositif d'application 1 et propre à recevoir du produit à distribuer. En particulier, la cavité de distribution 92 est formée dans la tête d'application 3 et est en communication fluidique avec au moins un orifice de distribution 91. Nous apercevons plus précisément sur les figures 6 et 7 que la première électrode 17 et la deuxième électrode 18 sont agencées au-dessus de la surface supérieure 5 de l'embase 4. La première électrode 17 et la deuxième électrode 18 sont reliées à un générateur de courant électrique (décrit plus loin dans la description) de sorte à produire un courant électrique qui est destiné à provoquer la pénétration du produit à distribuer dans la peau. La première électrode 17 et la deuxième électrode 18 pour iontophorèse sont séparées entre elles par une zone inter-électrode 19 assurant un espacement constant entre la première et la deuxième électrodes 17, 18. La zone inter-électrode 19 comprend une surface d'application 20 destinée à être en contact avec la peau pour appliquer le produit à distribuer. En d'autres termes, les premières et les deuxièmes électrodes 17, 18 sont en retrait de ladite zone inter-électrode 19, si bien que lesdites premières et les deuxièmes électrodes ne sont pas en contact avec la peau de l'utilisateur. De manière plus précise, la zone inter-électrode 19, est avantageusement, mais non limitativement, formée par un organe d'assemblage ayant une base 21 présentant une paroi latérale 22 reliant une première surface 23 orientée vers la peau de l'utilisateur, laquelle définit ainsi la surface d'application 20 et une deuxième surface 24 qui en est opposée. L'organe d'assemblage présente ici deux pions 25 qui s'étendent depuis la deuxième surface 24 et qui sont destinés à s'emmancher dans deux trous 26 correspondants de l'embase 4 de manière à permettre l'emmanchement de l'organe d'assemblage dans l'embase 4. Les trous 26 traversent l'embase 4 depuis la surface supérieure 5 vers la surface inférieure 6 de l'embase 4 (cf. figure 4). La paroi latérale 22 présentant une certaine épaisseur, la surface d'application 20 est située à distance de la surface supérieure 5 de l'embase 4. En particulier, la surface supérieure 5 de l'embase 4 est définie dans un plan A alors que les première et deuxième électrodes 17, 18, étant disposées au-dessus de la surface supérieure 5, sont définies dans un plan B parallèle au plan A. Quant à la zone inter-électrode 19, et en particulier, la surface d'application 20, celle-ci est définie dans un plan C qui est parallèle au plan B des première et deuxième électrodes 17, 18. Le plan B des première et deuxième électrodes 17, 18 et le plan C de la zone inter-électrode 19 sont situés à une distance prédéterminée l'un de l'autre. La distance prédéterminée est comprise entre 0.3 et 1.3 millimètres (mm). Suivant une caractéristique particulière de l'invention, la zone inter-électrode 19 présente une distance dmin minimale prédéterminée entre la première électrode 17 et la deuxième électrode 18. Cette distance dmin minimale prédéterminée est avantageusement comprise entre 5 mm et 20 mm. De préférence, mais non limitativement, cette distance dmin est de 10 mm. La cavité de distribution 92 est ainsi obtenue du fait de la distance séparant le plan B des première et deuxième électrodes 17, 18 et le plan C de la zone inter-électrode 19. Celle-ci présente donc au moins un fond formé par la surface supérieure 5 de l'embase 4 et des parois latérales formées par la paroi latérale 22 de l'organe d'assemblage (zone inter-électrode 19). Les parois latérales de la cavité 92 peuvent être formées également par une paroi interne d'une collerette 38 d'un capuchon 37 que comprend la tête d'application 3 qui est décrit ci-après. La cavité 92 de la tête d'application 3 permet qu'une fine pellicule de produit à distribuer se forme dans cette cavité 92. En d'autres termes, l'accumulation de produit est évitée sur l'interface supérieure de la tête d'application.

De manière avantageuse, mais non limitativement, le moyen de distribution 11 comprend un ou plusieurs orifices de sortie du produit vers la peau de l'utilisateur, lequel débouche sur l'interface supérieure de la tête d'application 3.

Selon une caractéristique de l'invention, la première électrode 17 et la deuxième électrode 18 présentent chacune une paroi 27 munie de perforations 28.

Suivant un premier mode de réalisation illustré sur la figure 6, la ou les perforations 28 distribuent le produit vers la peau de l'utilisateur. En d'autres termes, les perforations 28 constituent la ou les orifices de sortie du produit vers la peau. Ainsi, le produit circule à travers l'orifice de distribution 91 de l'embase 4 vers les perforations 28 de chaque première et deuxième électrodes 17, 18. Dans ce mode de réalisation, les première et deuxième électrodes sont réalisées dans un matériau électriquement conducteur. Ce matériau électriquement conducteur peut comprendre un matériau métallique ou un polymère ou un matériau composite comprenant ce matériau polymère. Le polymère peut être un polytétrafluoroéthylène (PTFE). Le matériau métallique peut être un acier inoxydable.

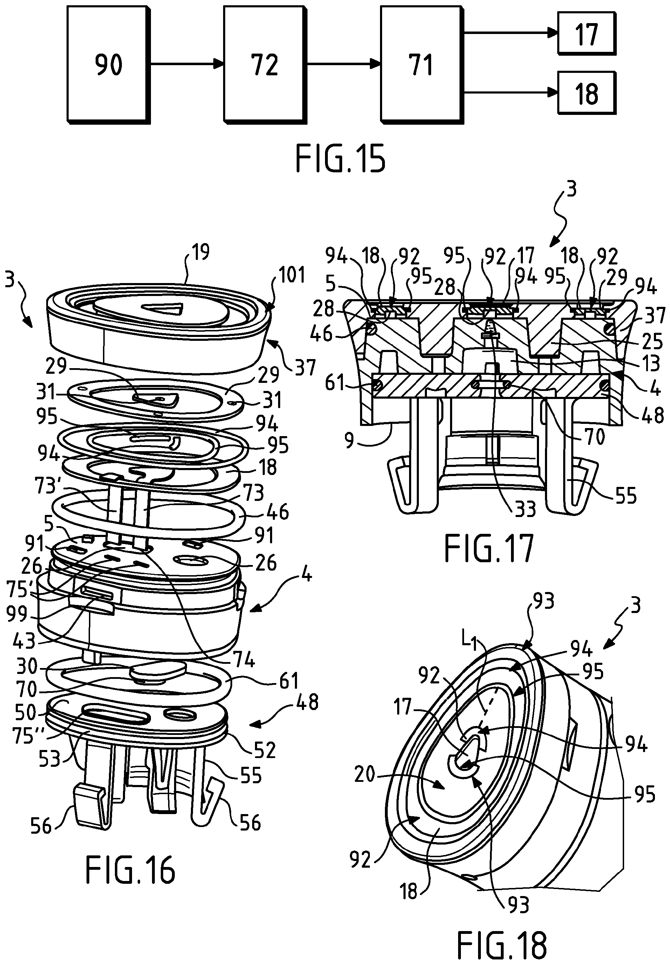

Suivant un deuxième mode de réalisation illustré sur la figure 7, ainsi qu'un autre mode de réalisation illustré sur la figure 16, au moins une plaque 29 présentant une paroi munie de perforations 31 est disposée dans la tête 3 d'application du dispositif 1 d'application. Plus précisément, au-dessus de chaque première et deuxième électrodes 17, 18 est agencée la plaque 29. Dans ce cas de figure, le produit circule à travers la ou les orifice(s) de distribution 91, la ou les perforations 28 des première et deuxième électrodes 17, 18 vers les perforations 31 de la plaque 29 lesquelles distribuent le produit vers la peau de l'utilisateur. De manière avantageuse, cette plaque 29 est non conductrice de sorte à éviter les irritations ou picotements sur la peau de l'utilisateur. La plaque 29 peut être réalisée dans un matériau polymère. De préférence, mais non limitativement, le polymère est un thermoplastique tel qu'un polytétrafluoroéthylène (PTFE) ou un polyoxyméthylène (POM). Dans ce mode de réalisation, les première et deuxième électrodes 17, 18 pour iontophorèse sont réalisées dans un matériau composite. De manière avantageuse, le matériau composite peut être un polymère chargé en carbone.

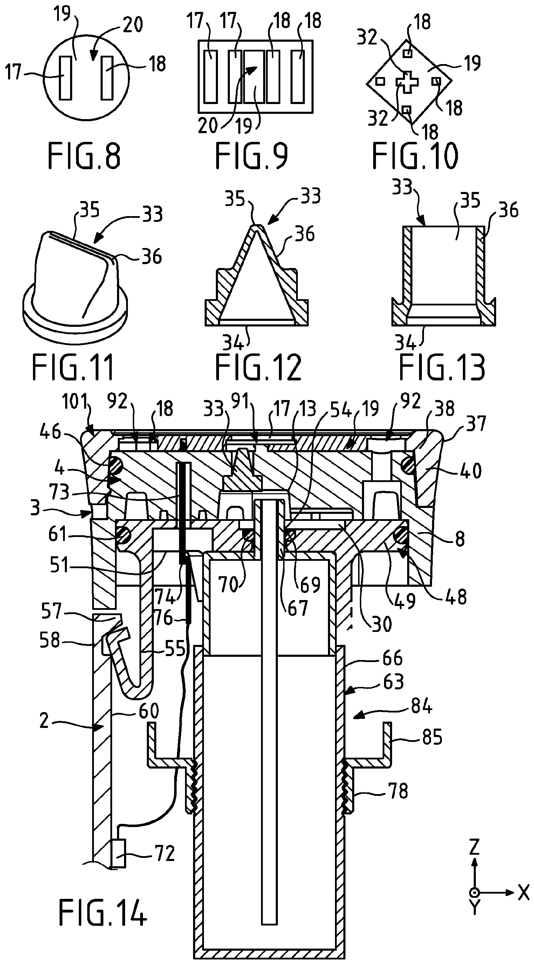

Les premières et deuxièmes électrodes 17, 18 peuvent être agencées de diverses manières au-dessus de la surface supérieure 5 de l'embase 4. Dans un mode de réalisation, tel qu'illustré sur les figures 1, 3, 6, et 7, la première électrode 17 est disposée au centre de l'embase 4. Quant à la deuxième électrode 18, celle-ci est disposée à la périphérie de la tête d'application 3, et de manière plus précise, autour du périmètre de la zone inter-électrode 19. La première électrode 17 illustrée, présente une forme sensiblement triangulaire. La deuxième électrode 18 présente, dans ce mode de réalisation, un contour fermé avec une forme générale triangulaire. Dans un autre mode de réalisation, illustré sur la figure 8, les première et deuxième électrodes 17, 18 pour iontophorèse présentent une forme rectangulaire. Celles-ci sont disposées parallèlement et sont séparées par la zone inter-électrode 19. Selon encore un autre mode réalisation illustré sur la figure 9, les première et deuxième électrodes 17, 18 sont de forme rectangulaire. Celles-ci sont au nombre de quatre et sont disposées à distance l'une de l'autre. La zone inter-électrode 19 est disposée au centre de la tête d'application 3 de sorte à avoir, par exemple, deux premières électrodes 17 à gauche de la zone inter-électrode 19 et deux deuxièmes électrodes 18 à droite de la zone inter-électrode 19. Selon encore un autre moyen de réalisation illustré sur la figure 10, la première électrode 17 présente une forme générale en croix présentant deux branches perpendiculaires formant alors quatre bords 32. La première électrode 17 est disposée au centre de la tête 3, et en particulier au centre de la surface supérieure 5 de l'embase 4. Des deuxièmes électrodes 18, ici quatre, sont disposées à distance de la première électrode 17 et autour de celle-ci. Chaque deuxième électrode 18 est disposée dans le prolongement d'un bord 32 de la première électrode 17. La zone inter-électrode 19 sépare la première et les deuxièmes électrodes 17, 18. D'autres configurations des première et deuxièmes électrodes 17, 18 sont, bien entendu, envisageables.

Dans les divers modes de réalisation décrits, la tête 3 d'application comprend au moins un moyen d'isolation 33 électrique qui est interposé fluidiquement entre la première électrode 17 et la deuxième électrode 18. Ce moyen d'isolation 33 électrique est configuré de manière à autoriser ou limiter, voire interdire le passage du courant électrique entre la première électrode 17 et la deuxième électrode 18 via la tête d'application 3. En référence aux figures 6 et 7, le moyen d'isolation 33 est disposé sur un des canaux de distribution 16. Afin de pouvoir contrôler la circulation du courant électrique entre la première électrode 17 et la deuxième électrode 18, le moyen d'isolation 33 électrique est disposé sur le canal de distribution 16 qui mène vers la première électrode 17, entre l'orifice de distribution 91 et la sortie 15 du réservoir tampon 13. En effet, sur les figures 6 et 7, la première électrode 17 pour iontophorèse est située au centre de la tête d'application 3 alors que la deuxième électrode 18 pour iontophorèse entoure la périphérie de la première électrode 17, et en particulier, la zone inter-électrode 19.

De manière avantageuse, mais non limitativement, le moyen d'isolation 33 électrique est situé à proximité de l'orifice de distribution 91 du canal de distribution 16 menant vers la première électrode 17 afin de limiter au maximum les courants de fuite vers la deuxième électrode 18 via la tête d'application 3.

Pour autoriser ou limiter le passage du courant entre la première électrode 17 et la deuxième électrode 18 via la tête d'application 3, le moyen d'isolation 33 électrique est apte à occuper une position ouverte et une position fermée. Dans la position fermée, le courant circule uniquement entre la première électrode 17 et la deuxième électrode 18. Dans la position ouverte, le courant circule de manière limitée dans la tête d'application 3 et dans le corps 2. Le moyen d'isolation 33 électrique présente une section de passage, qui, lorsque le moyen d'isolation 33 électrique occupe la position ouverte, permet la circulation du courant électrique entre la première et la deuxième électrodes 17, 18 et dans les canaux de distribution 16 de la tête d'application 3. Néanmoins, cette section de passage est tellement petite que l'impédance du chemin à parcourir est trop importante pour que la circulation du courant soit privilégiée via cette section de passage. Ainsi, le courant électrique circule quasi exclusivement dans l'interface supérieure (coté peau) plutôt que vers l'intérieur de la tête d'application 3 et le corps 2.

Dans la position ouverte, le produit à distribuer circule depuis le corps 2 vers les première et deuxième électrodes 17, 18 alors que dans la position fermée le produit ne circule plus vers la première électrode 17. Dès que du produit est de nouveau extrait depuis le moyen de stockage 84, vers le réservoir tampon 13, la pression du produit provoque l'ouverture du moyen d'isolation 33 électrique de manière à ce que les première et deuxièmes électrodes 17, 18 pour iontophorèse soient alimentées en produit. La pression d'ouverture du moyen d'isolation 33 électrique est choisie de manière adaptée par rapport aux canaux de distribution 16 alimentant la deuxième électrode 18 et la perte de charge associée. Idéalement, la perte de charge subie par le produit à distribuer au passage du moyen d'isolation 33 est égale à la perte de charge des canaux de distribution 16 plus longs alimentant la deuxième électrode 18. De manière alternative, des moyens d'isolation 33 électrique peuvent être également disposés sur les canaux de distribution 16 menant vers la deuxième électrode 18 pour iontophorèse. Cela permet avantageusement d'équilibrer la circulation du produit vers les première et deuxième électrodes 17, 18 pour iontophorèse, et ainsi de pouvoir optimiser l'égalité d'alimentation en produit des première et deuxième électrodes 17, 18 pour iontophorèse.

Par ailleurs, le moyen d'isolation 33 prend la position ouverte lorsqu'une pression à l'intérieur du moyen d'isolation 33 atteint un seuil prédéterminé. De manière avantageuse, mais non limitativement, le moyen d'isolation 33 est un clapet, et de préférence, un clapet de non retour tel qu'illustré sur les figures 11 à 13. Cependant, le moyen d'isolation 33 peut prendre une autre forme. Le clapet comprend un corps présentant une première extrémité au niveau de laquelle est formée une entrée 34 et une deuxième extrémité opposée au niveau de laquelle est formée une sortie 35. Cette dernière 35 se présente sous la forme d'un bec de canard formée de deux lèvres 36 opposées et élastiquement déformables. Les deux lèvres 36 s'ouvrent si la valeur de pression à l'intérieur du clapet est au moins égale à une valeur seuil de pression prédéterminée et se ferment quand la valeur de pression à l'intérieur du clapet est inférieure à la valeur seuil de pression prédéterminée. La valeur seuil de pression prédéterminée est comprise entre 5 et 200 millibars (mbar). De préférence, mais non limitativement, la valeur seuil de pression prédéterminée est de l'ordre de 50 mbar. Il est à noter que dans la position ouverte, la section de passage des lèvres 36 formée au niveau de la sortie 35 est très faible si bien que l'impédance est élevée. La section de passage maximale (d'ouverture) des lèvres 36 est comprise entre 0,2 mm² et 2,5 mm². Ainsi, la circulation du produit à distribuer est possible mais celle du courant est limitée entre la première électrode 17 et la deuxième électrode 18 pour iontophorèse via la tête d'application 3.

Le clapet est réalisé dans un matériau polymère, par exemple un silicone.

La tête 3 d'application comporte le capuchon 37 ou couvercle solidarisé à l'embase 4 pour maintenir les première et deuxième électrodes 17, 18 en position dans la tête d'application 3. Le capuchon 37 comprend la collerette 38 qui présente une ouverture centrale 39 par laquelle l'embase 4 est reçue. Les première et deuxième électrodes 17, 18 ainsi que l'organe d'assemblage (surface d'application 20) sont visibles à travers cette ouverture centrale 39. La collerette 38 présente une surface supérieure 101 qui est définie dans un plan D. Cette surface 101 supérieure forme un anneau d'appui qui a pour but de créer un volume avec une faible épaisseur sur la peau. Ce plan D est sensiblement parallèle au plan C de la surface d'application 20 de la zone inter-électrode 19. Le plan D est également parallèle au plan B des première et deuxième électrodes 17, 18 pour iontophorèse. La collerette 38 du capuchon 37 comprend à sa périphérie une jupe latérale 40 destinée à coopérer avec la paroi 7 de l'embase 4. En particulier, entre la jupe latérale 8 et la paroi 7 de l'embase 4 est prévu un épaulement 41 sur lequel prend appui une extrémité libre 42 de la jupe latérale 40. La paroi 7 de l'embase 4 présente également des encoches 43 aveugles (cf. figures 5 et 16) dans chacune desquelles est clippée une saillie 44 prévue sur une paroi interne 45 de la jupe latérale 40 pour fixer le capuchon 37 sur l'embase 4. Pour faciliter le démontage du couvercle 37 de l'embase 4, celle-ci comprend sur la paroi 7 une entaille 99. Cette dernière est située à proximité de chaque encoche 43 aveugle et permet qu'un doigt d'un utilisateur accède à l'extrémité libre 42 du couvercle 37.

Entre la paroi interne 45 de la jupe latérale 40 du capuchon 37 et la paroi 7 de l'embase 4 est agencé un premier joint d'étanchéité 46. Afin que le premier joint d'étanchéité 46 soit maintenu en position, la paroi 7 de l'embase 4 comprend une gorge 47 s'étendant dans le sens du périmètre de la paroi 7. Le premier joint d'étanchéité 46 possède un corps annulaire. Avantageusement, le premier joint d'étanchéité 46 est réalisé dans un matériau déformable élastiquement. Ce matériau déformable est de préférence un polymère ou un copolymère choisi parmi un éthylène-propylène-diène monomère (EPDM), un caoutchouc fluorocarboné (FPM), un élastomère polyacrylique(ACM), un copolymère éthylène acrylique (AEM), un caoutchouc nitrile hydrogéné (HNBR), un VITON ®, ou encore un butyle.

La tête d'application 3 comporte en outre un organe de connexion 48 pour connecter la tête 3 d'application et le corps 2 de manière amovible (cf. figures 5 et 16). Cet organe de connexion 48 comprend une partie de support 49 laquelle comprend une face supérieure 50 et une face inférieure 51 reliées par une paroi 52. La partie de support 49 présente une lumière 54 traversant la paroi 52 de part et d'autre. Cette lumière 54 communique avec l'entrée 14 du réservoir tampon 13. La partie de support 49 est équipée de pattes de fixation 55, ici il y en a trois, qui s'étendent depuis la face inférieure 51 de la partie de support 49. La partie de support 49 s'emboite à force avec la surface interne 9 de la jupe latérale 8 de l'embase 4. Les pattes de fixation 55 présentent vers chacune de leur extrémité libre 56 une forme sensiblement en V. Ces pattes de fixation 55 coopèrent avec un renflement 57 prévu à une extrémité proximale 58 d'une première portion 59 du corps 2. Le renflement 57 est agencé sur une surface interne 60 du corps 2. La paroi 52 est pourvue d'une gorge 53 s'étendant le long du périmètre de la paroi 52 et dans laquelle est installé un second joint d'étanchéité 61. Ce dernier 61 est en contact avec la surface interne 9 de la jupe latérale 8.

De manière avantageuse, le produit à distribuer est contenu dans une cartouche 63 qui est connectée de manière amovible au corps 2 du dispositif. Ainsi, lorsque le produit est épuisé, la cartouche 63 peut être remplacée facilement ou être à nouveau remplie.

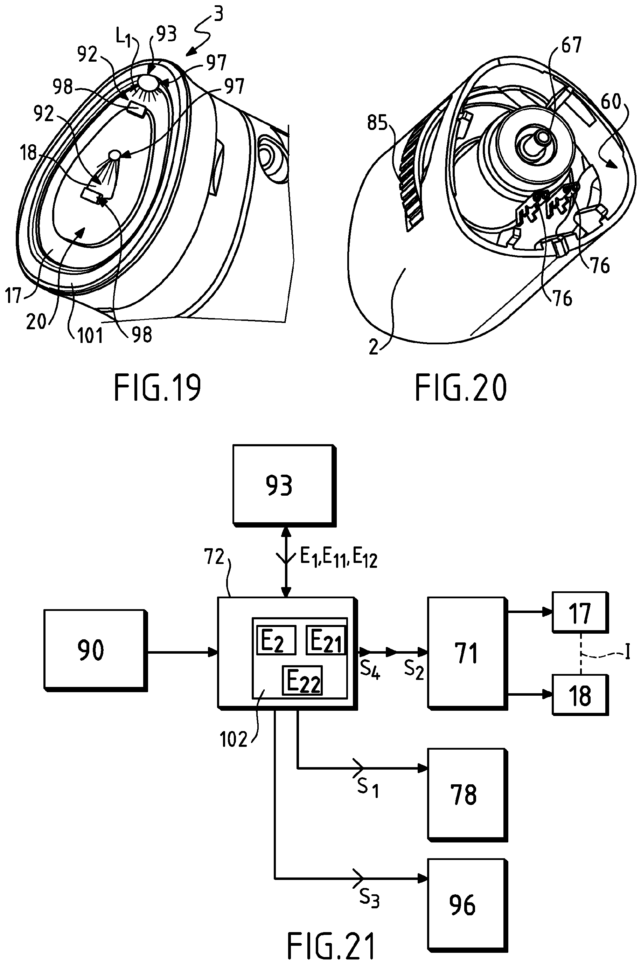

En référence aux figures 14 et 20, la cartouche 63 comprend un boitier 66 recevant une tête de fixation 67 La tête de fixation 67 est introduite à travers la lumière 54 de l'organe de connexion 48 et une portion de celle-ci s'étend dans le réservoir tampon 13. La tête de fixation 67 est fixe par rapport à la tête d'application 3. Un joint d'étanchéité 70 est également logé dans une gorge 69 de la partie de support 49 de l'organe de connexion 48. Le joint d'étanchéité 70 est plaqué dans la gorge 69 par une butée 30 de sorte que le produit à distribuer ne coupe pas la tête de fixation 67 de la cartouche 63.

Le corps 2 comprend au moins un moyen d'extraction 78 permettant d'extraire le produit du moyen de stockage 84 depuis le corps 2. Ce moyen d'extraction 78 peut être manuel ou motorisé. En mode manuel, l'appareil peut comprendre un moyen d'entrainement 82 comprenant une portion 83 (cf. figure 1) s'étendant à travers l'ouverture 77 du corps 2 du dispositif 1 de sorte que l'utilisateur l'actionne facilement. De manière avantageuse, mais non limitativement, ce moyen d'entrainement 82 comprend une molette 85.

En variante, le moyen d'extraction 78 motorisé comprend un moteur (non représenté) actionnant des moyens permettant d'entrainer en rotation le mécanisme de distribution. Le moteur peut être actionné via un bouton (non représenté) accessible sur le corps 2 du dispositif 1 d'application. Ce bouton est relié à un circuit électronique de commande 72 pour actionner le moteur.

Dans le corps 2 est également agencé un générateur de courant 71 permettant de délivrer un courant de faible intensité aux première et deuxième électrodes 17, 18 disposées dans la tête 3 d'application. Le courant électrique est compris entre 50 microampères (µA) et 6000 µA. De préférence, mais non limitativement le courant électrique est de l'ordre de 800 µA. Le courant peut être alternatif ou continu. Le générateur de courant 71 électrique est commandé par le circuit électronique de commande 72 installé dans le corps 2 du dispositif 1 d'application de produit pour fournir le courant. Le circuit électronique de commande 72 est alimenté par une source d'alimentation 90 électrique pouvant être une pile ou une batterie rechargeable sur un réseau électrique domestique. Ceci est illustré dans un schéma bloc à la figure 15. De manière alternative, le dispositif 1 peut comporter un cordon d'alimentation électrique (non représenté) destiné à être branché sur le réseau électrique domestique et à alimenter le circuit électronique de commande 72. Afin d'assurer une connexion électrique entre le générateur de courant 71 et les première et deuxième électrodes 17, 18, ces dernières sont dotées chacune d'une broche 73, 73' (cf. figures 5, 14 et 16). Les broches 73, 73' et les première et deuxième électrodes 17, 18 sont formées ici d'une seule pièce (d'un seul tenant). Chaque broche 73, 73' (cf. figures 14 et 16) présente une extrémité libre 74 accessible depuis l'interface inférieure de la tête d'application 3. En d'autres termes, chaque broche 73, 73' traverse la zone inter-électrode 19, l'embase 4 et l'organe de connexion 48 de part et d'autre via des fentes 75, 75', 75" que comprennent ceux-ci. Les broches 73, 73' sont donc accessibles depuis la face inférieure 51 de l'organe de connexion 48 de la tête d'application 3. Chaque broche 73, 73' présente une longueur supérieure à celles de la zone inter-électrode, de l'embase 4 et de l'organe de connexion 48. Ici, chacune des broches 73, 73' présente une section en forme de L inversé.Le corps 2 du dispositif comprend des éléments de connexion 76 qui sont reliés au générateur de courant 71. Un élément de connexion 76 est connecté à l'anode du générateur de courant 71 et un autre élément de connexion 76 est connecté à la cathode du générateur de courant 71. De la sorte, lorsque la tête d'application 3 est connectée avec le corps 2 du dispositif 1, chaque broche 73, 73' coopère avec un élément de connexion 76 et est alimentée en courant électrique.

Selon une caractéristique de l'invention, lorsque du produit est distribué, un circuit fermé est formé avec la peau de l'utilisateur, la première électrode 17 pour iontophorèse et la deuxième électrode 18 pour iontophorèse. Afin d'éviter des microlésions de la peau créant alors des sensations d'inconfort, voire de douleurs en cas de forte intensité ou de durée prolongée, ou pour détecter une anomalie de fonctionnement ou de tolérance, le dispositif peut comprendre une sonde de température. De manière avantageuse, cette sonde de température est intégrée dans la tête d'application 3 du dispositif d'application 1 et reliée au circuit électronique de commande 72.

Dans un autre mode de réalisation tel qu'illustré sur les figures 16 à 19 et 21, le dispositif 1 d'application comprend des moyens de mesure 93 d'un paramètre physique ou physico-chimique reliés au circuit électronique de commande 72. Ces moyens de mesure 93 sont configurés de manière à envoyer au circuit électronique de commande 72 un signal de mesure E₁ de paramètre. Dans ce mode de réalisation, le circuit électronique de commande 72 est configuré pour comparer la valeur mesurée du paramètre physique ou physico-chimique à une valeur seuil de référence de façon à détecter s'il y a absence de produit dans la cavité de distribution 92. De manière avantageuse mais non limitativement, le circuit électronique de commande 72 comprend une mémoire 102 dans laquelle est stockée la valeur seuil de référence. Le circuit électronique de commande 72 comprend également un comparateur (non représenté) destiné à comparer la valeur seuil avec la valeur mesurée par les moyens de mesure 93.

Les moyens de mesure 93 comprennent, suivant un premier mode de réalisation illustré sur les figures 16 à 18, un moyen de mesure d'impédance dans la cavité de distribution 92. Ce moyen de mesure d'impédance est localisé dans au moins une cavité de distribution 92. Ici, ce moyen de mesure d'impédance comporte une paire d'électrodes disposées dans chaque cavité de distribution 92 et entre lesquelles peut circuler un courant électrique. Chaque paire d'électrodes comprend une première électrode émettrice 94 propre à émettre un courant électrique I₁ et une deuxième électrode réceptrice 95 propre à recevoir le courant électrique I₁ émis par la première électrode émettrice 94. La première électrode émettrice 94 et la deuxième électrode réceptrice 95 sont connectées à un générateur de tension (non représenté). Chacune des première électrode émettrice 94 et deuxième électrode réceptrice 95 est réalisée par surmoulage lors de la fabrication de l'organe d'assemblage 19 et/ou du capuchon 37 afin de faciliter le montage de celles-ci. Il peut être bien entendu possible de réaliser les électrodes par un procédé de sérigraphie ou autres méthodes similaires. De manière avantageuse, les première et deuxième électrodes 17, 18 pour iontophorèse et les électrodes 94, 95 pour la mesure d'impédance sont indépendantes. Les électrodes 94, 95 pour la mesure d'impédance sont réalisées dans le ou les même(s) matériaux que les première et deuxième électrodes 17, 18 pour iontophorèse. La première électrode émettrice 94 est disposée en regard de la deuxième électrode réceptrice 95. Le courant électrique I₁ est transmis par la première électrode émettrice 94 à la deuxième électrode réceptrice 95. Puis la valeur de l'intensité de ce courant électrique I₁ est convertie en une valeur d'impédance correspondante mesurée. Cette valeur d'impédance à l'intérieur de la cavité de distribution 92 correspond, ici, à la valeur du signal de mesure E₁. Le signal de mesure E₁ est transmis au circuit électronique de commande 72 lequel compare la valeur du signal de mesure E₁ à une valeur d'impédance seuil E₂ prédéterminée. De manière avantageuse, le circuit électronique de commande 72 est destiné à générer au moins un signal de commande S1, S2, S3 en fonction du signal de mesure E₁.

De manière avantageuse, mais non limitativement, quand la valeur du signal de mesure E₁ mesurée est inférieure ou égale à une valeur d'impédance seuil prédéterminée E₂ alors le circuit électronique de commande 72 génère un signal de commande S1, S2, S3. A l'inverse, lorsque la valeur du signal de mesure E₁ mesurée est supérieure à la valeur d'impédance seuil E₂ prédéterminée, le circuit de commande 72 n'envoie pas de signal de commande. En particulier, le circuit électronique de commande 72 envoie un signal de commande S1, S2, S3 lorsqu'il y a absence de produit dans la cavité de distribution 92. Tandis que lorsque le circuit électronique de commande 72 n'envoie pas de signal de commande, alors il y a présence de produit dans la cavité 92. Bien entendu, le circuit électronique de commande 72 peut être configuré pour générer un signal de commande s'il y a présence de produit dans la cavité 92. Celui-ci peut par exemple émettre un signal de commande S2 pour réactiver l'alimentation électrique des première et deuxième électrodes 17, 18 pour iontophorèse.

Ainsi, lorsqu'il y a absence de produit dans la cavité de distribution 92, le circuit électronique de commande 72 envoie les signaux de commandes suivants :
- un signal de commande S1 propre à déclencher la distribution de produit dans la cavité de distribution 92 ;
- un signal de commande S2 destiné à générer un courant électrique propre à circuler dans les première et deuxième électrodes 17, 18 pour iontophorèse ; ou
- un signal de commande S3 propre à avertir l'utilisateur de l'absence de produit dans la cavité de distribution 92.

Dans le cas du signal de commande S1, celui-ci active le moteur actionnant les moyens d'entrainement de la cartouche 63 dans le corps 2 de l'appareil.

Dans le cas du signal de commande S2, celui-ci réactive le générateur de courant 71 pour générer un courant électrique dans les première et deuxième électrodes 17, 18 pour iontophorèse. En effet, lorsque la mesure est effectuée dans la cavité de distribution 92 par chaque paire d'électrodes de mesure d'impédance, les première et deuxième électrodes 17, 18 pour iontophorèse ne sont pas actives. Pour cela, le circuit électronique de commande 72 est configuré pour envoyer un signal de commande S4 pour couper l'alimentation électrique des première et deuxième électrodes 17, 18 pour iontophorèse durant la mesure.

Dans le cas du signal de commande S3, le dispositif 1 d'application comprend des moyens d'alerte 96 connectés au circuit électronique de commande 72 pour avertir l'utilisateur de l'absence de produit dans la cavité 92. Les moyens d'alerte 96 peuvent être des moyens visuels et/ou sonores. L'utilisateur ainsi alerté décidera en conséquence si le traitement ou soin de la peau est terminé auquel cas il n'actionnera pas le moyen d'extraction 78 du produit à distribuer. Dans le cas contraire, l'utilisateur actionnera le moyen d'extraction 78.

Les moyens de mesure 93 comprennent, suivant un deuxième mode de réalisation illustré sur la figure 19, un moyen de mesure optique. Ce moyen de mesure optique est également localisé dans la cavité de distribution 92 du produit à distribuer. Le moyen de mesure optique du produit permet de transmettre des informations représentatives de l'intensité lumineuse dans la cavité de distribution 92. Le moyen de mesure optique est connecté au circuit électronique de commande 72 lequel est apte à traiter les informations représentatives de l'intensité lumineuse dans la cavité de distribution 92. Pour cela, le moyen de mesure optique comporte au moins un émetteur 97 d'un rayon lumineux et au moins un récepteur 98 du rayon lumineux disposé en regard de l'émetteur de lumière 97. L'émetteur 97 présente une source de lumière propre à émettre un rayon lumineux L₁ et disposé d'un côté de la cavité de distribution 92. Cette source de lumière comprend par exemple une diode électroluminescente (siglé LED pour Light-Emitting Diode) qui émet des longueurs d'onde comprises entre 500 et 1500 nm. Le récepteur 98 présente un récepteur de lumière propre à recevoir le rayon lumineux L₁ émis par la source de lumière et est placé de l'autre côté de la cavité de distribution 92. Le récepteur 98 du rayon lumineux comprend une photopile recevant la ou les longueurs d'onde émise(s) par la LED et apte à le ou les transformer en un courant électrique. Dans ce mode de réalisation, le moyen de mesure optique effectue une première mesure afin de détecter si le dispositif est en contact ou non avec la peau de l'utilisateur. Dans le cadre de cette première mesure, le rayon lumineux L1 est transmis par l'émetteur 97 au récepteur 98. Puis, le rayon lumineux L₁ est converti en courant électrique représentatif de l'intensité lumineuse reçue. Cette valeur d'intensité lumineuse à l'intérieur de la cavité de distribution 92 correspond, ici, à la valeur du signal de mesure E₁₁. Le signal de mesure E₁₁ est transmis au circuit électronique de commande 72 lequel compare la valeur du signal de mesure E₁₁ à une valeur d'intensité lumineuse seuil E₂₁ prédéterminée. De manière avantageuse, le circuit électronique de commande 72 est destiné à générer un premier signal de commande ou un deuxième signal en fonction du signal de mesure E₁₁.

De manière avantageuse, mais non limitativement, quand la valeur du signal de mesure E₁₁ mesurée est inférieure à la valeur d'intensité lumineuse seuil E₂₁ prédéterminée alors le circuit électronique de commande 72 génère un premier signal de commande. A l'inverse, lorsque la valeur du signal de mesure E₁₁ mesuré est supérieure ou égale à la valeur d'intensité lumineuse seuil E₂₁ prédéterminée le circuit de commande 72 envoie un deuxième signal de commande. En particulier, le circuit électronique de commande 72 envoie le premier signal de commande lorsque le dispositif est en contact avec la peau de l'utilisateur et le deuxième signal de commande lorsque le dispositif n'est pas en contact avec la peau.

Lorsque le circuit électronique de commande 72 détecte que le dispositif 1 est en contact avec la peau, alors le circuit électronique de commande envoie au moyen de mesure optique le premier signal de commande propre à déclencher une deuxième mesure. Cette deuxième mesure permet de détecter s'il y a absence de produit dans la cavité de distribution 92. Pour cela, un signal de mesure E₁₂ représente une valeur d'intensité lumineuse dans la cavité de distribution 92. Le signal de mesure E₁₂ est transmis au circuit électronique de commande 72 lequel compare la valeur du signal de mesure E₁₂ à une valeur d'intensité lumineuse seuil E₂₂ prédéterminée. De manière avantageuse, le circuit électronique de commande 72 est destiné à générer un signal de commande S1, S2, S3 en fonction du signal de mesure E₁₂. Lorsque la valeur du signal de mesure E₁₂ mesurée est inférieure ou égale à la valeur d'intensité lumineuse seuil E₂₂ prédéterminée, alors le circuit électronique de commande 72 ne génère pas de signal de commande. A l'inverse, lorsque la valeur du signal de mesure E₁₂ mesurée est supérieure à la valeur d'intensité lumineuse seuil E₂₂ prédéterminée, le circuit de commande 72 génère un signal de commande S1, S2, S3. En particulier, le circuit électronique de commande 72 émet un signal de commande S1, S2, S3 lorsqu'il y a absence de produit dans la cavité de distribution 92. Tandis que lorsque le circuit électronique de commande 72 n'émet pas de signal de commande, alors il y a présence de produit dans la cavité 92. Bien entendu, le circuit électronique de commande 72 peut être configuré pour générer un signal de commande s'il a présence de produit dans la cavité 92. Ainsi, lorsqu'il y a absence de produit dans la cavité de distribution 92, le circuit électronique de commande 72 envoie les signaux de commandes suivants. Ces signaux de commandes sont identiques aux signaux de commande générés lors de la mesure d'impédance.
- un signal de commande S1 propre à déclencher la distribution de produit dans la cavité de distribution 92 ;
- un signal de commande S2 destiné à générer un courant électrique propre à circuler dans les première et deuxième électrodes 17, 18 pour iontophorèse ; ou,
- un signal de commande S3 propre à avertir l'utilisateur de l'absence de produit dans la cavité de distribution 92 ;

Nous allons maintenant décrire le fonctionnement du dispositif 1 d'application d'un produit à distribuer sur la peau d'un utilisateur par iontophorèse. Lorsque le dispositif 1 d'application est mis sous tension, le circuit électronique de commande 72 adresse un ordre au générateur de courant 71 lequel génère un courant électrique I aux première et deuxième électrodes 17, 18 pour iontophorèse. L'utilisateur actionne le moyen d'extraction 78 jusqu'à ce que le produit soit extrait de la cartouche 63 et collecté dans un premier temps dans le réservoir tampon 13. Dans un deuxième temps, le produit sous pression déclenche l'ouverture du clapet, lorsque la pression à l'intérieur de celui atteint une valeur prédéterminée, ce qui permet le passage du produit dans les canaux de distribution 16 formés dans l'embase 4. Le produit débouche alors dans la cavité de distribution 92 via les orifices de distribution 91 vers les première et deuxième électrodes 17, 18 pour iontophorèse. Le produit traversant les perforations 28 des première et deuxième électrodes 17, 18 pour iontophorèse au droit du champ électrique créé est transporté en profondeur de la peau suivant les lignes de champs formant un arc en demi-cercle. La distance atteinte sous la peau est comprise entre 2 et 10 mm de profondeur.

Suivant un mode de réalisation de l'invention, le dispositif effectue des mesures de paramètres physique ou physico-chimiques dans la cavité de distribution 92 afin de déterminer s'il y a absence de produit dans la cavité de distribution 92 du dispositif 1. Ces mesures peuvent être programmées pour avoir lieu après une période de temps prédéterminée pendant le fonctionnement du dispositif 1. Dans un premier temps, le circuit électronique de commande 72 envoie un signal de commande S4 pour couper l'alimentation électrique des première et deuxième électrodes 17, 18 pour iontophorèse. Puis le circuit électronique de commande 72 déclenche le procédé de détection lequel présente les étapes suivantes :
- mesure par les moyens de mesure 93 d'une valeur d'un paramètre physique ou physico-chimique dans la cavité de distribution 92 ;
- transmission de cette valeur mesurée au circuit électronique de commande 72 ;
- comparaison, par le circuit électronique de commande 72, de cette valeur mesurée de paramètre physique ou physico-chimique avec une valeur seuil de référence E₂, E₂₁, E₂₂; et,
- détection, par le circuit électronique de commande 72, de l'absence de produit dans la cavité de distribution 92.

Lorsqu'il est détecté qu'il y a absence de produit dans la cavité de distribution 92 alors le circuit électronique de commande 72 génère un signal de commande S3 pour avertir l'utilisateur de l'absence de produit dans la cavité. L'utilisateur prend la décision ou non d'actionner le moyen d'extraction 78.

De manière alternative, lorsqu'il est détecté qu'il y a absence de produit dans la cavité de distribution 92 alors le circuit électronique de commande 72 génère un signal de commande tel qu' :
- un signal de commande S1 propre à déclencher la distribution de produit dans la cavité de distribution 92 ; et/ou,
- un signal de commande S2 destiné à générer un courant électrique propre à circuler dans les première et deuxième électrodes 17, 18 pour iontophorèse.

L'invention est décrite dans ce qui précède à titre d'exemple. Il est entendu que la personne de l'art est à même de réaliser différentes variantes de réalisation de l'invention, en associant par exemple les différentes caractéristiques ci-dessus prises seules ou en combinaison, sans pour autant sortir du cadre de l'invention.

## Revendications

1. Dispositif (1) d'application d'un produit à distribuer sur une peau d'un utilisateur par iontophorèse comprenant:
- au moins un moyen de stockage (84) du produit à distribuer;
- au moins une cavité de distribution (92) propre à recevoir du produit à distribuer ;
- au moins une première électrode (17) pour iontophorèse ;
- un circuit électronique de commande (72);
- des moyens de mesure (93) d'un paramètre physique ou physico-chimique reliés au circuit électronique de commande (72) et configurés de manière à envoyer un signal (E₁, E₁₁, E₁₂) indicatif de mesure de paramètre,
**caractérisé en ce que** la première électrode pour iontophorèse (17) et les moyens de mesures sont installés dans la cavité de distribution (92), le circuit électronique de commande (72) étant configuré pour comparer la valeur mesurée du paramètre physique ou physico-chimique dans la cavité à une valeur seuil de référence (E₂, E₂₁, E₂₂) de façon à détecter s'il y a absence du produit dans la cavité (92).

2. Dispositif (1) selon la revendication 1, **caractérisé en ce qu'**il comprend au moins une deuxième électrode (18) pour iontophorèse agencée dans la cavité de distribution (92).

3. Dispositif (1) selon la revendication 1 ou 2, **caractérisé en ce que** le circuit électronique de commande (72) est configuré, s'il détecte l'absence de produit dans la cavité de distribution, pour générer un signal de commande (S1) propre à déclencher la distribution de produit dans la cavité de distribution (92).

4. Dispositif (1) selon la revendication 1 ou 2, **caractérisé en ce qu'**il comprend des moyens d'alerte (96) connectés au circuit électronique de commande (72), le circuit électronique de commande (72) étant configuré, s'il détecte l'absence de produit dans la cavité de distribution (92), pour générer un signal de commande (S3) propre à avertir l'utilisateur de l'absence de produit dans la cavité (92).

5. Dispositif selon l'une quelconque des revendications précédentes, **caractérisé en ce qu'**il comprend au moins un moyen d'extraction (78) du produit à distribuer à partir du moyen de stockage (84).

6. Dispositif selon l'une quelconque des revendications précédentes, **caractérisé en ce que** les moyens de mesure (93) comprennent un moyen de mesure d'impédance du produit.

7. Dispositif selon la revendication 6, **caractérisé en ce que** le moyen de mesure d'impédance comprend au moins une paire d'électrodes pour la mesure d'impédance, disposée dans la cavité de distribution (92) et entre lesquelles circule un courant électrique (I₁).

8. Dispositif selon l'une quelconque des revendications 1 à 5, **caractérisé en ce que** les moyens de mesure (93) comprennent un moyen de mesure optique.

9. Dispositif (1) selon la revendication 8, **caractérisé en ce que** le moyen de mesure optique comprend au moins un émetteur (97) de lumière diffusant une lumière à travers au moins une des cavités de distribution (92) et au moins un récepteur (98) de lumière placé en regard de l'émetteur (97).

10. Dispositif (1) selon l'une quelconque des revendications précédentes, **caractérisé en ce que** la première électrode et la deuxième électrode (17, 18) pour iontophorèse sont séparées par une zone inter-électrode (19) laquelle comprend une surface d'application (20) du produit sur la peau.

11. Dispositif (1) selon l'une quelconque des revendications précédentes, **caractérisé en ce que** les première et deuxième électrodes (17, 18) pour iontophorèse sont situées dans un même plan (B).

12. Dispositif (1) selon la revendication 11 lorsqu'elle dépend de la revendication 10, **caractérisé en ce que** la surface d'application (20) est située dans un plan (C) parallèle audit plan (B) des première et deuxième électrodes (17, 18), le plan (B) des première et deuxième électrodes pour iontophorèse et le plan (C) de la zone inter-électrode (19) étant situés à une distance prédéterminée.

13. Procédé de détection de l'absence de produit à distribuer dans une cavité de distribution (92) d'un dispositif (1) d'application du produit selon l'une quelconque des revendications 1 à 12, le procédé comprenant les étapes suivantes :
- mesure par les moyens de mesure (93) d'une valeur d'un paramètre physique ou physico-chimique dans la cavité de distribution (92) ;
- transmission de cette valeur mesurée au circuit électronique de commande (72) ;
- comparaison, par le circuit électronique de commande 72, de cette valeur mesurée de paramètre physique ou physico-chimique avec une valeur seuil de référence (E₂, E₂₁, E₂₂); et,
- détection, par le circuit électronique de commande 72, de l'absence de produit dans la cavité de distribution (92) .

## Patentansprüche

1. Vorrichtung (1) zur Applikation eines auf eine Haut eines Anwenders abzugebenden Produkts durch lontophorese, umfassend:
- mindestens ein Lagerungsmittel (84) des abzugebenden Produkts;
- mindestens einen zum Aufnehmen des abzugebenden Produkts geeigneten Abgabehohlraum (92);
- mindestens eine erste Elektrode (17) für lontophorese;
- einen elektronischen Steuerungsschaltkreis (72);
- Messmittel (93) eines physikalischen oder physiko-chemischen Parameters, verbunden mit dem elektronischen Steuerungsschaltkreis (72) und auf eine Weise konfiguriert, um ein für die Parametermessung indikatives Signal (E₁, E₁₁, E₁₂) zu senden,
**dadurch gekennzeichnet, dass** die erste Elektrode für lontophorese (17) und die Mittel der Messungen in dem Abgabehohlraum (92) installiert sind, wobei die elektronische Steuerungsschaltung (72) konfiguriert ist, um den gemessenen Wert des physikalischen oder physiko-chemischen Parameters in dem Hohlraum mit einem Referenz-Schwellenwert (E₂, E₂₁, E₂₂) auf eine Art zu vergleichen, um zu Detektieren, ob Produkt in dem Hohlraum (92) abwesend ist.

2. Vorrichtung (1) nach Anspruch 1, **dadurch gekennzeichnet, dass** sie mindestens eine in dem Abgabehohlraum (92) angeordnete zweite Elektrode (18) für lontophorese umfasst.

3. Vorrichtung (1) nach Anspruch 1 oder 2, **dadurch gekennzeichnet, dass** die elektronische Steuerungsschaltung (72) konfiguriert ist, um, falls sie die Abwesenheit des Produkts in dem Abgabehohlraum detektiert, ein Steuerungssignal (S1) zu erzeugen, das geeignet ist, die Abgabe des Produkts in den Abgabehohlraum (92) auszulösen.

4. Vorrichtung (1) nach Anspruch 1 oder 2, **dadurch gekennzeichnet, dass** sie Warnmittel (96) umfasst, verbunden mit der elektronischen Steuerungsschaltung (72), wobei die elektronische Steuerungsschaltung (72) konfiguriert ist, um, falls sie die Abwesenheit des Produkts in dem Abgabehohlraum (92) detektiert, ein Steuerungssignal (S3) zu erzeugen, das geeignet ist, den Anwender über die Abwesenheit des Produkts in dem Hohlraum (92) zu benachrichtigen.

5. Vorrichtung nach einem der vorstehenden Ansprüche, **dadurch gekennzeichnet, dass** sie mindestens ein Extraktionsmittel (78) des ausgehend von dem Lagerungsmittel (84) abzugebenden Produkts umfasst.

6. Vorrichtung nach einem der vorstehenden Ansprüche, **dadurch gekennzeichnet, dass** die Messmittel (93) ein Messmittel der Impedanz des Produkts umfassen.

7. Vorrichtung nach Anspruch 6, **dadurch gekennzeichnet, dass** das Messmittel der Impedanz mindestens ein Paar von Elektroden für die Messung der Impedanz umfasst, die in dem Abgabehohlraum (92) angebracht sind und zwischen denen ein elektrischer Strom (I₁) fließt.

8. Vorrichtung nach einem der Ansprüche 1 bis 5, **dadurch gekennzeichnet, dass** die Messmittel (93) ein optisches Messmittel umfassen.

9. Vorrichtung (1) nach Anspruch 8, **dadurch gekennzeichnet, dass** das optische Messmittel mindestens einen Lichtemitter (97), der ein Licht durch mindestens einen der Abgabehohlräume (92) streut, und mindestens einen dem Emitter (97) zugewandt platzierten Lichtempfänger (98) umfasst.

10. Vorrichtung (1) nach einem der vorstehenden Ansprüche, **dadurch gekennzeichnet, dass** die erste Elektrode und die zweite Elektrode (17, 18) für lontophorese durch eine Zwischenelektrodenzone (19) getrennt sind, die eine Oberfläche zur Applikation (20) des Produkts auf die Haut umfasst.

11. Vorrichtung (1) nach einem der vorstehenden Ansprüche, **dadurch gekennzeichnet, dass** die erste und zweite Elektrode (17, 18) für lontophorese sich in einer gleichen Ebene (B) befinden.

12. Vorrichtung (1) nach Anspruch 11, wenn sie von Anspruch 10 abhängt, **dadurch gekennzeichnet, dass** die Oberfläche zur Applikation (20) sich in einer Ebene (C) parallel zur Ebene (B) der ersten und zweiten Elektrode (17, 18) befindet, wobei die Ebene (B) der ersten und zweiten Elektrode für lontophorese und die Ebene (C) der Zwischenelektrodenzone (19) in einem vorher festgelegten Abstand gelegen sind.

13. Verfahren zur Detektion der Abwesenheit eines abzugebenden Produkts in einem Abgabehohlraum (92) einer Vorrichtung (1) zur Applikation des Produkts nach einem der Ansprüche 1 bis 12, wobei das Verfahren die folgenden Schritte umfasst:
- Messung eines Werts eines physikalischen oder physiko-chemischen Parameters in dem Abgabehohlraum (92) durch die Messmittel (93);
- Übermittlung dieses gemessenen Werts an die elektronische Steuerungsschaltung (72);
- Vergleich dieses gemessenen Werts des physikalischen oder physiko-chemischen Parameters mit einem Referenz-Schwellenwert (E₂, E₂₁, E₂₂) durch die elektronische Steuerungsschaltung (72); und
- Detektion der Abwesenheit des Produkts in dem Abgabehohlraum (92) durch die elektronische Steuerungsschaltung (72).

## Claims

1. Device (1) for applying a product to be distributed on the skin of a user by iontophoresis comprising:
- at least one means of storage (84) for storing the product to be distributed;
- at least one distribution cavity (92) able to receive the product to be distributed;
- at least one first electrode (17) for iontophoresis;
- an electronic control circuit (72);
- means for measuring (93) a physical or physicochemical parameter connected to the electronic control circuit (72) and configured to send a signal (E₁, E₁₁, E₁₂) indicating the parameter measurement,
**characterised in that** the first electrode for iontophoresis (17) and the means for measuring are installed in the distribution cavity (92), the electronic control circuit (72) being configured to compare the measured value of the physical or physicochemical parameter in the cavity with a threshold reference value (E₂, E₂₁, E₂₂) so as to detect an absence of product in the cavity (92).

2. Device (1) according to claim 1, **characterised in that** it comprises at least one second electrode (18) for iontophoresis arranged in the distribution cavity (92).

3. Device (1) according to claim 1 or 2, **characterised in that** the electronic control circuit (72) is configured, if it detects the absence of product in the distribution cavity, to generate a command signal (S1) able to trigger distribution of product in the distribution cavity (92).

4. Device (1) according to claim 1 or 2, **characterised in that** it comprises means of alert (96) connected to the electronic control circuit (72), the electronic control circuit (72) being configured, if it detects the absence of product in the distribution cavity (92), to generate a command signal (S3) able to alert the user to the absence of product in the cavity (92).

5. Device (1) according to any of the preceding claims, **characterised in that** it comprises at least one means of extraction (78) of the product to be distributed from the means of storage (84).

6. Device according to any of the preceding claims, **characterised in that** the means for measuring (93) comprise means for measuring the impedance of the product.

7. Device according to claim 6, **characterised in that** the means for measuring impedance comprises at least one pair of electrodes for the measurement of impedance, arranged in the distribution cavity (92) and between which an electric current (I₁) circulates.

8. Device according to any of claims 1 to 5, **characterised in that** the means for measuring (93) comprise a means for optical measurement.

9. Device (1) according to claim 8, **characterised in that** the means for optical measurement comprise at least one light transmitter (97) diffusing a light through at least one of the distribution cavities (92) and at least one light receiver (98) placed opposite the transmitter (97).

10. Device (1) according to any of the preceding claims, **characterised in that** the first electrode and the second electrode (17, 18) for iontophoresis are separated by an inter-electrode zone (19) which comprises a surface of application (20) of the product on the skin.

11. Device (1) according to any of the preceding claims, **characterised in that** the first and second electrodes (17, 18) for iontophoresis are situated in the same plane (B).

12. Device (1) according to claim 11, when it depends on claim 10, **characterised in that** the surface of application (20) is situated in a plane (C) parallel to said plane (B) of the first and second electrodes (17, 18), the plane (B) of the first and second electrodes for iontophoresis and the plane (C) of the inter-electrode zone (19) being situated at a predetermined distance.

13. Process for detecting the absence of product to be distributed in a distribution cavity (92) of a product application device (1) according to any of claims 1 to 12, the method comprising the following steps:
- measuring via the means for measuring (93) of a value of a physical or physicochemical parameter in the distribution cavity (92);
- transmitting this measured value to the electronic control circuit (72);
- comparing, by the electronic control circuit (72), this measured value of the physical or physicochemical parameter with a threshold reference value (E₂, E₂₁, E₂₂); and,
- detecting, by the electronic control circuit (72), the absence of product in the distribution cavity (92).
